# EUROPÄISCHE PATENTSCHRIFT

(11) **EP 0 947 207 B1**
(45) Veröffentlichungstag und Bekanntmachung des Hinweises auf die Patenterteilung: **03.03.2004**
(21) Anmeldenummer: 99810270.1
(22) Anmeldetag: 30.03.1999
(51) Int. Cl.: A61M 5/148

(54) **Pulsationsfreie Fördervorrichtung für sterile Flüssigkeiten**
Non pulsating delivery device for sterile fluids
Dispositif d'administration sans pulsations pour fluides stériles

(30) Priorität: 31.03.1998 CH 75298
(43) Veröffentlichungstag der Anmeldung: 06.10.1999
(73) Patentinhaber: Nisco Engineering AG, 8008 Zürich (CH)
(72) Erfinder: Brandenberger, Harry, 8006 Zürich (CH)
(74) Vertreter: Clerc, Natalia

(56) Entgegenhaltungen:
- EP-A- 0 343 286
- EP-A- 0 676 214
- WO-A-94/11054
- WO-A-94/27659
- FR-A- 2 592 306
- US-A- 2 842 123
- US-A- 3 199 511
- US-A- 4 332 246

## Beschreibung

Aus der US-A-3,199,511 ist eine Infusionsvorrichtung zur Behandlung von Patienten bekannt zum Fördern einer Flüssigkeit zu einer Injektionsnadel. Sie umfasst einen abschliessbaren Behälter. Im Deckel des Behälters ist eine Öffnung vorgesehen, in welche ein Zapfen eingesteckt ist. Auf der Innenseite des Behälters ist an den Zapfen ein Beutel angeschlossen, der die Infusionsflüssigkeit enthält und über einen Anschluss am Zapfen mit einem Schlauch kommuniziert. Das Schlauchende ist an die Infusionsnadel angeschlossen. Der den Beutel umgebende Innenraum des Behälters ist mit einer Flüssigkeit gefüllt, die über eine Dosierpumpe zugeführt wird. Dadurch kann die durch die Nadel zugeführte Flüssigkeit mit einer Durchflussrate von 0,05 bis 10 cm³/min geregelt werden.

Diese Vorrichtung eignet sich zum Zuführen relativ kleiner Gesamtmengen.

EP-A-0'343'286 betrifft eine Fördervorrichtung, welche einem Patienten eine Flüssigkeit zuführt. Hier ist ein Behälter mit einer extrem dehnbaren Membran zur Aufnahme eines Beutels offenbart, welcher mit der abzugebenden Flüssigkeit gefüllt ist. Der Behälter wird sich über eine Pumpe mit einem Medium gefüllt, wobei sich die Luft im Behälter über eine Lüftungsöffnung absaugen lässt. Der Behälter ist in einer Ausführungsform zweiteilig ausgebildet und kann wie ein Buch geöffnet werden, um den Beutel in die Membran einzulegen. In einer zweiten Ausführungsform ist der Behälter ohne Membran ausgebildet, so dass der Beutel direkt mit dem Medium beaufschlagt wird.

Auch US-A-4'332'246 und WO 94/27659 betreffen Fördervorrichtungen zur Zuführung von Flüssigkeiten zu einem Patienten. In US'246 hängt der Beutel in einem Behälter und wird von einer Flüssigkeit beaufschlagt, in WO'659 ist der Beutel von einem Gas umgeben und liegt auf dem Boden auf. Der vorliegenden Erfindung liegt die Aufgabe zugrunde, eine Vorrichtung der eingangs genannten Art anzugeben, die über einen grösseren Bereich der Gesamtmenge der zu dosierenden Flüssigkeit einsetzbar ist.

Die Aufgabe wird mittels einer Vorrichtung gelöst, wie sie im unabhängigen Patentanspruch definiert ist. Besonders geeignete Ausführungsformen sind in den abhängigen Patentansprüchen definiert.

Weiter wird ein Pumpaggregat vorgeschlagen, an das ein oder mehrere erfindungsgemässe Fördervorrichtungen angeschlossen sind, und somit der Pumpvorgang autonom und flexibel durchgeführt werden kann. Insbesondere das im Patentanspruch 9 definierte Pumpaggregat ist wirtschaftlich interessant, da eine Pumpe für verschiedene Medien verwendet werden kann.

Im folgenden werden anhand der Figuren einzelne Ausführungsbeispiele bzw. Einsatzschritte der erfindungsgemässen Vorrichtung näher beschrieben. Die Figuren enthalten dabei:
- Figur 1: Bereitstellen der Verbindungseinheit,
- Figur 2: Verbinden des Beutels mit der autoklavierten Verbindungseinheit und Füllen des Beutels, und
- Figur 3: Einbau des Beutels und pulsationsfreie Förderung.

Eine Zellsuspension soll möglichst pulsationsfrei in einen Bioreaktor gefördert werden.

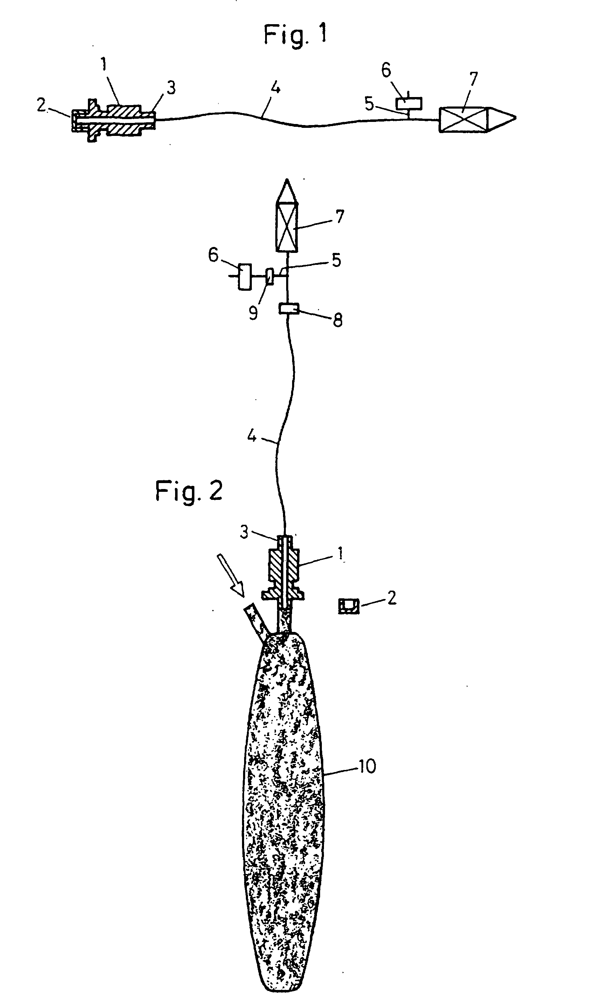

Die als Adapter 1 ausgebildeten Mittel zum Anschliessen des Beutels werden mit dem Blinddeckel 2 verschlossen und mittels sterilem Schlauchverbindungsstück 3 mit dem Schlauch 4 mit eingebautem T-Stück 5 und Sterilluftfilter 6 verbunden. Auf der anderen Seite des Schlauches wird das geeignete sterile Verbindungsstück 7 (Anstecheinheit oder geeignetes autoklavierbares Ventil) befestigt. Die in Figur 1 dargestellte Vorrichtung wird als Verbindungseinheit im Autoklaven sterilisiert.

Das Verbinden der autoklavierten Verbindungseinheit mit dem Beutel ist in Figur 2 dargestellt. Dazu wird zuerst im "laminar air bench" die autoklavierte Verbindungseinheit mit dem Beutel 10 verbunden, indem der Blinddeckel 2 entfernt und die Verbindung erstellt wird. Der Beutel 10 wird in aufrechter Lage mit der Zellsuspension gefüllt, wobei der Beutel durch den Schlauch 4 über den Sterilfilter 6 entlüftet wird. Die Flüssigkeit steigt über den Adapter 1 in den Schlauch 4 bis zur Klemmvorrichtung 8.

Hat die Flüssigkeit diese Klemmvorrichtung erreicht, so wird diese geschlossen. Danach wird die Klemmvorrichtung 9 geschlossen und der Filter 6 entfernt. Beutel mit Verbindungseinheit bilden jetzt eine Sterileinheit und können in den Produktionsraum transportiert werden.

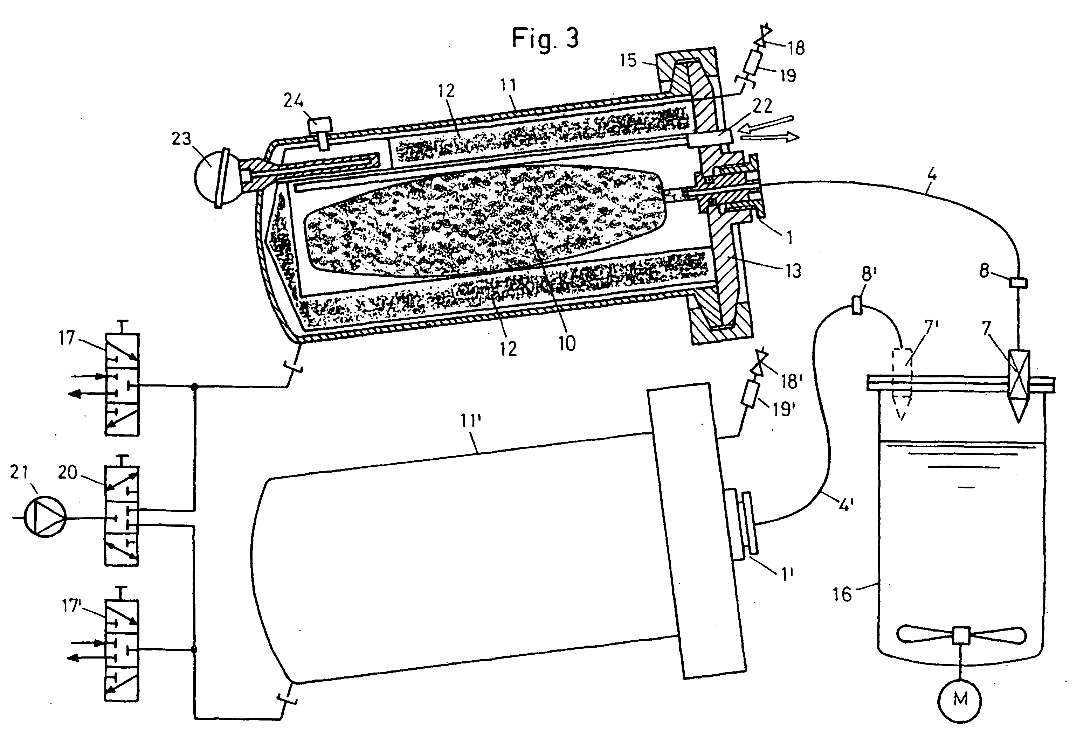

Figur 3 zeigt das Anschliessen der Vorrichtung an der Anlage und Durchführen der pulsationsfreien Förderung.

Nun kann im Produktionsraum der steril abgeschlossene Beutel in einen Behälter 11 eingeführt werden, der ein oder mehrere inkompressible Verdrängungselemente 12 enthält zur Definition der Beutellage und Verkleinerung des freien Volumens. Die Verbindungseinheit wird durch die Öffnung am Deckel 13 eingeführt und danach der Adapter 1 mit der Fixierungsvorrichtung 14 fixiert. Der Adapter dichtet mittels O-Ring-Dichtung ab. Mit der Clamp-Verbindung 15 wird der Deckel auf den Behälter befestigt. Durch das Verbindungsstück 7, in der für den Bioreaktor entsprechenden Ausführung, wird das System an den Reaktor 16 angeschlossen.

Jetzt wird entgastes Wasser über das Ventil 17 in den Behälter gefüllt. Über ein Entlüftungsventil 18 wird der Behälter entlüftet. Nachdem der letzte Rest Luft aus dem Behälter durch das Wasser verdrängt ist (im Schauglas 19 erkennbar), wird das Ventil 17 geschlossen und das Ventil 20 geöffnet. Ein Förderorgan 21, beispielsweise eine präzise Doppelkolbenpumpe, fördert entgastes Wasser in den Behälter. Nach der Füllstandskontrolle am Schauglas 19 wird das Entlüftungsventil 18 geschlossen, so dass eine Druckkraft auf den Beutel wirkt.

Jetzt kann der unterbrochene Zuführschlauch 4 mittels Klemmvorrichtung 8 geöffnet werden, was in einem konstanten Volumenstrom in den Reaktor 16 resultiert: Das gleiche Volumen Wasser, das unsteril in den Behälter gepumpt wird, kommt als Flüssigkeitsvolumen aus dem sterilen Beutel in den Reaktor.

Temperaturempfindliche Zellsuspensionen können während der Zugabe mittels einer Kühleinheit 22 gekühlt werden. Eine Temperaturmessung 23 kann integriert werden.

Sobald der Beutel leer ist, entsteht ein markanter Druckanstieg im Behälter. Das Abschalten der Pumpe kann über Drucksensoren 24 automatisiert werden. Jetzt kann batchweise ein neuer Beutel eingeführt werden.

Wie in Figur 3 dargestellt ist, kann nebst dem einen Behälter 11 ein zweiter Behälter 11' bereitgestellt werden, der gleich ausgebildet ist, wobei der in ihm enthaltene Beutel ebenfalls mit einer Verbindungseinheit 1', 4', 7' mit dem Reaktor 16 verbunden ist. Der zweite Behälter 11' wird ebenfalls über ein Ventil 17' mit Wasser gefüllt. Wenn der Beutel 10 im ersten Behälter 11 leer ist, kann die Pumpe 21 über das Ventil 20 auf den zweiten Behälter 11' umgeschaltet werden, so dass die Zudosierung der sterilen Flüssigkeit ohne Unterbruch fortgesetzt werden kann. Das Wasser im ersten Behälter 11 wird dann nach dem Öffnen des Ventils 18 über das Ventil 17 entleert, der Deckel 13 demontiert und der leere Beutel 10 mit Verbindungseinheit 1, 4, 7 gegen eine neue Einheit ausgetauscht. Danach kann der Behälter 11 in der beschriebenen Weise für den nächsten Fördervorgang vorbereitet werden.

Je nach Anwendung kann der Beutel im zweiten Behälter 11' auch eine andere Flüssigkeit enthalten. In diesem Fall kann es zweckmässig sein, für diesen Behälter 11' eine separate Pumpe 21 bereitzustellen.

Das Verfahren zum Fördern einer sterilen Flüssigkeit in einem sterilen Prozessraum mit der erfindungsgemässen Fördervorrichtung besteht aus folgenden Schritten:
1. Autoklavierung einer geeigneten Verbindungseinheit und Anschluss an einen sterilen Beutel.
2. Füllen des sterilen Beutels mit einer Flüssigkeit unter sterilen Bedingungen.
3. Verschliessen der Anschlüsse und Einführen des Beutels in einen festen Behälter.
4. Schliessen des Behälters mit einer Verschlussvorrichtung.
5. Verbinden mit einem sterilen Prozessraum über die Verbindungseinheit.
6. Vollständiges Füllen des Behälters mit entgaster Flüssigkeit.
7. Öffnen der Verbindung zum sterilen Prozessraum.
8. Förderung von entgaster Flüssigkeit in den Behälter, folglich Förderung aus dem sterilen Beutel in den sterilen Prozessraum.
9. Wenn der Beutel leer ist (Druckanstieg im Behälter):
   a) Umstellen auf ein paralleles Fördersystem mittels 3-Weg-Ventilen zur quasistationären Förderung, oder
   b) Öffnen des Behälters und Auswechseln des Beutels.

Das Verfahren zur Entnahme einer sterilen Flüssigkeit aus einem sterilen Prozessraum mit der erfindungsgemässen Fördervorrichtung besteht aus folgenden Schritten:
1. Autoklavierung einer geeigneten Verbindungseinheit und Anschluss an einen sterilen Beutel.
2. Verschliessen der Anschlüsse und Einführen des Beutels in einen festen Behälter.
3. Schliessen des Behälters mit einer Verschlussvorrichtung.
4. Verbinden mit einem sterilen Prozessraum über die Verbindungseinheit.
5. Vollständiges Füllen des Behälters mit entgaster Flüssigkeit.
6. Öffnen der Verbindung zum sterilen Prozessraum.
7. Förderung von entgaster Flüssigkeit aus dem Behälter, folglich Förderung in den sterilen Beutel aus dem sterilen Prozessraum.
8. Wenn der Beutel voll ist:
   a) Umstellen mittels 3-Weg-Ventilen auf einen parallelen Beutel, oder
   b) Öffnen des Behälters und Entnahme des Beutels.

Vorteile dieser Fördervorrichtung sind:
1. Eine Flüssigkeit kann mit einfachen Mitteln pulsationsfrei in einen sterilen Raum gefördert werden.
2. Die Messung des Volumenstroms ist über Durchflussmesser oder Messung in der Pumpe sehr einfach.
3. Raumbedarf und Kontaminationsgefahr werden minimiert.
4. Es können beliebige Dosier- oder Entnahmekurven angefahren oder ein konstanter Strom gefahren werden.
5. Weil der Beutel 10 im Behälter 11 durch das Verdrängungselement 12 gestützt ist und der zylindrische Behälter 11 im wesentlichen liegend oder leicht geneigt angeordnet ist, wird dessen Verbindung mit dem Adapter 1 auch vor dem Einfüllen des Wassers nicht belastet. Dadurch können Beutel 10 mit wesentlich grösserem Fassungsvolumen eingesetzt werden als bei der eingangs genannten Vorrichtung. Die liegende Anordnung hat zudem den Vorteil, dass der Beutelinhalt vollständiger entleert werden kann als bei hängender Anordnung, vor allem auch bei unterschiedlichem spezifischem Gewicht der beiden Flüssigkeiten.
6. Durch die Verdrängungselemente 12 kann das einzufüllende Wasservolumen minimiert werden. Dadurch wird eine rasche Inbetriebnahme erreicht auch bei stark variierendem Fassungsvermögen der Beutel 10.
7. Durch Zuschalten des zweiten Behälters 11' ist eine praktisch ununterbrochene Zudosierung der Flüssigkeit möglich.

## Patentansprüche

1. Fördervorrichtung zur sterilen und pulsationsfreien Förderung von Flüssigkeiten von einem oder in einen Prozessraum, umfassend einen gegen die Umgebung abschliessbaren Behälter (11), wobei erste Mittel (1, 4, 7) zum Anschliessen eines im Innenraum des Behälters (11) anzuordnenden Beutels (10) vorhanden sind, welcher dazu bestimmt ist, die Flüssigkeit aufzunehmen oder abzugeben und über die ersten Mittel (1, 4, 7) an den externen Prozessraum (16) pulsationsfrei abzugeben oder von ihm aufzunehmen, wobei der Innenraum über zweite Mittel (20) mit einer Pumpe (21) verbunden ist, welche dazu bestimmt ist, ein die Aussenseite des Beutels (10) direkt beaufschlagendes, steriles oder nicht steriles, inkompressibles Medium in den oder aus dem Innenraum zu fördern, **dadurch gekennzeichnet, dass** im Behälter (11) mindestens ein Stützelement für den Beutel (10) angeordnet ist.

2. Vorrichtung nach Anspruch 1, wobei der Innenraum mindestens ein auswechselbares, imkompressibles Einsatzelement (12) enthält, das das Aufnahmevolumen für das von der Pumpe förderbare Medium reduziert, wobei der Behälter (11) vorzugsweise einen abnehmbaren Deckel (13) hat.

3. Vorrichtung nach Anspruch 2, wobei das Stützelement entweder als Teil des Einsatzelementes (12) ausgebildet oder von diesem unabhängig ist.

4. Vorrichtung nach einem der Ansprüche 1 bis 3, wobei sie einen Thermostaten (23) und/oder einen Drucksensor (24) im Bereich des von der Pumpe (21) förderbaren Mediums aufweist.

5. Vorrichtung nach einem der Ansprüche 1 bis 4, wobei der Behälter (11) zylindrisch ausgebildet und liegend oder leicht geneigt angeordnet ist.

6. Vorrichtung nach einem der Ansprüche 1 bis 5, wobei die Mittel (1, 4, 7) zum Anschliessen des Beutels (10) steril bzw. sterilisierbar sind und vorzugsweise über eine lösbare Fixiereinrichtung (14) am Behälter (11) oder dessen Deckel (13) anbringbar sind.

7. Vorrichtung nach einem der Ansprüche 1 bis 7, wobei das Medium eine entgaste Flüssigkeit, insbesondere Wasser ist.

8. Pulsationsfreies Pumpaggregat enthaltend eine Fördervorrichtung nach einem der Ansprüche 1 bis 7, wobei der Behälter ein erster Behälter (11) ist, wobei mindestens ein zweiter Behälter (11') angeordnet ist, in dem dritte Mittel (1', 4', 7') zum Anschliessen eines weiteren Beutels vorhanden sind, welcher dazu bestimmt ist, die Flüssigkeit aufzunehmen oder abzugeben und über die dritten Mittel an den externen Prozessraum (16) pulsationsfrei abzugeben oder von ihm aufzunehmen, wobei der Innenraum des zweiten Behälters (11') über das zweite Mittel (20) mit der Pumpe (21) oder über ein weiteres Mittel mit einer weiteren Pumpe verbunden ist, welche dazu bestimmt ist, ein mit der Flüssigkeit nicht in Berührung stehendes steriles oder nicht steriles Medium in den Innenraum des zweiten Behälters (11') zu fördern.

9. Pumpaggregat nach Anspruch 8, wobei es eine einzige Pumpe (21) enthält, die über das zweite Mittel (20) wahlweise mit dem Innenraum des ersten oder zweiten Behälters verbindbar ist.

10. Pumpaggregat nach Anspruch 9, wobei das Medium über einen Volumenstromregler zeitlich konstant oder programmgesteuert in den ersten und/oder zweiten Behälter bzw. alle Behälter einleitbar ist.

## Claims

1. Delivery device for delivering liquids in a sterile and non-pulsating fashion from or to a processing chamber, consisting of a container (11) sealed from its surroundings, whereby connecting means (1, 4, 7) for connecting a bag (10) to be placed in the interior of the container (11) are present, the former being intended to receive or discharge the liquid and to deliver or receive it non-pulsating to or from the external processing chamber (16) through a first set of elements (1, 4, 7), whereby the inner chamber is connected through a second set of elements (20) to a pump (21), used to deliver an incompressible, sterile or non-sterile medium which acts directly on the external side of the bag (10) to or from the inner chamber, **characterized in that** the container (11) has at least one supporting element for the bag (10).

2. Device according to claim 1, in which the inside contains at least one replaceable, incompressible insertable element (12) that reduces the intake volume of the medium delivered by the pump, and the container (11) preferably has a removable cover (13).

3. Device according to claim 2, in which the supporting element either forms one piece with the insertable element (12) or is independent from the same.

4. Device according to one of claims 1 to 3, provided with a thermostat (23) and/or a pressure sensor (24) in the space occupied by the medium delivered by the pump (21).

5. Device according to one of claims 1 to 4, in which the container (11) is cylindrical and placed in a horizontal or slightly inclined position.

6. Device according to one of claims 1 to 5, in which the elements (1, 4, 7) for connecting the bag (10) are sterile or can be sterilized, and can preferably be attached to the container (11) or its cover (13) by means of a removable locking device (14).

7. Device according to one of claims 1 to 7, in which the medium is a degassed liquid, in particular water.

8. Non-pulsating pump unit containing a delivery device according to one of claims 1 to 7, having a first container (11) and at least a second container (11') provided with a third set of elements (1', 4', 7') for connecting another bag, intended to receive or deliver the liquid and to deliver or receive it non-pulsating to or from the external processing chamber (16) through the third set of elements, whereby the interior of the second container (11') is connected by means of the second element (20) to the pump (21) or by means of another element to another pump, intended to deliver a sterile or non-sterile medium, which is not in contact with the liquid, to the interior of the second container (11').

9. Pump unit according to claim 8, containing a single pump (21), which optionally can be connected to the inside of the first or the second container by means of the second element (20).

10. Pump unit according to claim 9, in which the medium can be introduced into the first and/or second or all containers through a volumetric flow regulator in a time constant or program controlled fashion.

## Revendications

1. Dispositif d'administration pour l'administration stérile et sans pulsations de fluides depuis ou dans un espace de traitement, comprenant un récipient (11) pouvant être fermé par rapport à l'environnement, des premiers moyens (1, 4, 7) pour le raccordement d'un sachet (10) à disposer dans l'espace interne du récipient (11) étant prévus, le sachet étant prévu pour recevoir ou délivrer le fluide et à le délivrer par le biais des premiers moyens (1, 4, 7) à l'espace de traitement externe (16) ou à l'en prélever sans pulsations, l'espace interne étant connecté par le biais de deuxièmes moyens (20) à une pompe (21) qui est prévue pour refouler un milieu incompressible stérile ou non stérile, sollicitant directement le côté extérieur du sachet (10), dans ou depuis l'espace interne, **caractérisé en ce que** l'on dispose dans le récipient (11) au moins un élément de support pour le sachet (10).

2. Dispositif selon la revendication 1, dans lequel l'espace interne contient au moins un élément d'insert incompressible remplaçable (12), qui réduit le volume de réception pour le milieu pouvant être refoulé par la pompe, le récipient (11) ayant de préférence un couvercle amovible (13).

3. Dispositif selon la revendication 2, dans lequel l'élément de support est réalisé soit comme une partie de l'élément d'insert (12), soit est indépendant de celui-ci.

4. Dispositif selon l'une quelconque des revendications 1 à 3, dans lequel il présente un thermostat (23) et/ou un capteur de pression (24) dans la région du milieu pouvant être refoulé par la pompe (21).

5. Dispositif selon l'une quelconque des revendications 1 à 4, dans lequel le récipient (11) est cylindrique et est couché ou légèrement incliné.

6. Dispositif selon l'une quelconque des revendications 1 à 5, dans lequel les moyens (1, 4, 7) pour raccorder le sachet (10) sont stériles ou stérilisables et peuvent être disposés de préférence par le biais d'un dispositif de fixation détachable (14) sur le récipient (11) ou sur son couvercle (13).

7. Dispositif selon l'une quelconque des revendications 1 à 7, dans lequel le milieu est un fluide dégazé, en particulier de l'eau.

8. Ensemble de pompe sans pulsations contenant un dispositif d'administration selon l'une quelconque des revendications 1 à 7, dans lequel le récipient est un premier récipient (11), au moins un deuxième récipient (11') étant prévu, dans lequel des troisièmes moyens (1', 4', 7') pour le raccordement à un autre sachet sont prévus, le sachet étant prévu pour recevoir ou délivrer le fluide et pour le délivrer par le biais des troisièmes moyens à l'espace de traitement externe (16) ou à l'en prélever sans pulsations, l'espace interne du deuxième récipient (11') étant connecté par le biais du deuxième moyen (20) à la pompe (21) ou par le biais d'un autre moyen à une autre pompe, laquelle est prévue pour refouler un milieu stérile ou non stérile qui n'est pas en contact avec le fluide dans l'espace interne du deuxième récipient (11').

9. Ensemble de pompe selon la revendication 8, contenant une pompe unique (21), qui peut être connectée par le biais du deuxième moyen (20) au choix à l'espace interne du premier ou du deuxième récipient.

10. Ensemble de pompe selon la revendication 9, dans lequel le milieu peut être introduit dans le premier et/ou le deuxième récipient ou dans tous les récipients par le biais d'un régulateur de débit volumique de manière constante dans le temps ou de manière commandée par un programme.
